# EUROPÄISCHE PATENTSCHRIFT

(11) **EP 0 739 630 B1**
(45) Veröffentlichungstag und Bekanntmachung des Hinweises auf die Patenterteilung: **12.03.2003**
(21) Anmeldenummer: 96106691.7
(22) Anmeldetag: 26.04.1996
(51) Int. Cl.: A61K 35/14, A61M 1/38, C07K 14/525

(54) **Verfahren zur simultanen extrakorporalen Elimination von Tumor-Nekrose-Faktor alpha und bakteriellen Lipopolysacchariden aus Vollblut oder/und Blutplasma**
Process for the simultaneous elimination of tumor necrosis factor alpha and bacterial lipopolysaccharides from whole blood and/or blood plasma
Procédé pour l'élimination simultanée du facteur de nécrose tumorale alpha et de lipopolysaccharides bactériens à partir de sang complet et/ou de plasma sanguin

(30) Priorität: 27.04.1995 DE 19515554
(43) Veröffentlichungstag der Anmeldung: 30.10.1996
(73) Patentinhaber: B. BRAUN MELSUNGEN AG, 34212 Melsungen (DE)
(72) Erfinder: Boos, Karl-Siegfried, Prof. Dr., 82131 Gauting (DE); Seidel, Dietrich, Prof. Dr. med., 82340 Feldafing (DE); Trautwein, Annette, 67069 Ludwigshafen/Oppau (DE); Morsch, Gerold, 34628 Willinghausen (DE)
(74) Vertreter: Böhm, Brigitte, Dipl.-Chem. Dr.

(56) Entgegenhaltungen:
- EP-A- 0 592 989
- EP-A- 0 705 845
- DE-A- 2 815 811
- DE-A- 3 110 128
- DE-A- 4 209 988
- US-A- 3 664 506
- US-A- 4 059 512
- WEBER C ET AL: "DEVELOPMENT OF CATIONICALLY MODIFIED CELLULOSE ADSORBENTS FOR THE ENDOTOXINS" ASAIO JOURNAL, Bd. 41, Nr. 3, 1.Juli 1995, Seiten 430-434, XP000542926

## Beschreibung

Die vorliegende Erfindung betrifft ein Verfahren zur simultanen Entfernung von Tumor-Nekrose Faktor α (TNFα) und bakteriellen Lipopolysacchariden (LPS) aus Vollblut oder/und Blutplasma in einem extrakorporalen Perfusionssystem.

Die selektive und effektive Eliminierung des Tumor-Nekrose-Faktors (TNFα) und von bakteriellen Lipopolysacchariden (LPS, Synonym: Endotoxine) aus dem Blut bzw. Plasma von Patienten ist aus medizinischer Sicht für die Prävention und Therapie einer gram-negativen Sepsis erwünscht (Intensivtherapie bei Sepsis und Multiorganversagen, Schuster, H.-P., ed., 1993, Springer-Verlag, Berlin.) Die Prognose einer schweren Sepsis mit einhergehendem Schock ist unter der derzeitigen Standardtherapie schlecht.

Der septische Schock ist gekennzeichnet durch eine Fehlverteilung des Blutflusses bei einem gleichzeitigen drastischen Abfall des peripheren Widerstandes. In der akuten Phase findet sich eine Herzdilatation und die kardiale Auswurffraktion ist deutlich vermindert. Mit Progression des Krankheitsverlaufs tritt in rascher zeitlicher Abfolge ein Versagen von zwei oder mehr vitalen Organsystemen (Multi-Organversagen) als klinischer Endpunkt auf. Bei über 50 % dieser Intensivpatienten muß trotz aller therapeutischen Bemühungen mit einem letalen Ausgang gerechnet werden. In den USA wird die Anzahl der durch einen septischen Schock hervorgerufenen Todesfälle auf ca. 100 000 pro Jahr geschätzt (Parillo, J.E., "Septic Shock in Humans" in: Annals of Internal Medicine, Vol. 113, No. 3, 1990, 227-242).

Die septischen Komplikationen (Schock, Multi-Organversagen) enstehen durch gram-positive und/oder gram-negative Bakterien. Die Invasion der Bakterien in die Blutbahn führt bei grampositiven Bakterien (z.B. Staphylococcus aureus) zur Sezernierung von Exotoxinen, bei der Lyse von gram-negativen Bakterien (z.B. Escherichia coli) zur Freisetzung von LPS (Endotoxine) aus der äußeren Bakterienwand. Die bakteriellen Lipopolysaccharide besitzen eine stäbchenartige Form und sind aus drei strukturell unterschiedlichen Regionen aufgebaut. Der Träger der toxischen Eigenschaften ist das Lipid-A. Diese für nahezu alle Lipopolysaccharide invariable Substruktur besitzt ein Molekulargewicht von 2000 Dalton und besteht aus einem phosphorylierten D-Glucosamin-Disaccharid, an das ester- und amidartig mehrere langkettige Fettsäuren gebunden sind (Bacterial Endotoxic Lipopolysaccharides, Morrison, D.C., Ryan, J.L. eds., 1992, CRC Press).

In die Blutbahn eingeschwemmtes LPS bindet an die Zellen des Monozyten-Makrophagen-Systems und stimuliert diese zu einer gesteigerten Produktion und Freisetzung von Mediatoren (Cytokine). Als initialer Mediator und potenter proinflammatorischer Stimulus wird zunächst der Tumor-Nekrose-Faktor α synthetisiert und in die Blutbahn sezerniert. Die biologisch wirksame Form des TNF α besteht aus einem Aggregat dreier identischer Polypeptidketten (157 Aminosäuren, Molekulargewicht: 17,4 x 10³ Dalton; Ziegler, E.J., N. Engl. J. Med 318, 1988, 1533 ff). Die nachfolgende biologische Signalverstärkung über Interleukine, Leukotriene, Prostaglandine und Interferone (Mediatorkaskade) kann schließlich schwere Störungen der Homöostase verschiedener biologischer Regelkreise und Organsysteme, wie beispielsweise das Krankheitsbild des septischen Schocks, hervorrufen. So konnte gezeigt werden, daß das klinische Bild einer Sepsis in vielen Fällen mit dem Verlauf und der Höhe der LPS-Konzentration im Blut der Patienten korreliert (Nitsche, D. et al., Intensive Care Med., 12 Suppl., 1986, 185 ff). Darüber hinaus gibt es Hinweise, daß eine Korrelation zwischen der TNF α-Konzentration im Blutplasma und dem Grad der septischen Schock-Symptome bzw. dem späteren Todeseintritt besteht (Grau, G.E., et al., Immunol. Rev. 112, 1989, 49 ff). Somit nehmen die Lipopolysaccharide (LPS) als initiierende Toxine gram-negativer Bakterien und TNF α als initial freigesetzter Mediator eine Schlüsselrolle hinsichtlich der Pathogenese einer gram-negativen Sepsis ein.

Die derzeitige Therapie einer Sepsis beinhaltet neben den konventionellen intensiv-therapeutischen Maßnahmen beispielsweise die Gabe von speziellen Antibiotika (Shenep, I.L., Morgan, K.A., J. Infect. Dis. 150, 1984, 380 ff), von Immunglobulinen (Schedel, F. et al., Crit. Care Med. 19, 1991, 1104 ff.) oder von Antikörpern gegen LPS oder TNF α (Werdan, K., Intensivmed. 30, 1993, 201 ff.). Jedoch können auch diese Therapieschemata die Prognose (Überlebensrate) dieser - mit einer hohen Mortalität behafteten - Patientengruppe nicht wesentlich verbessern. Erste tierexperimentelle Studien weisen in diesem Zusammenhang darauf hin, daß bei einer gleichzeitigen Gabe von Antikörpern gegen LPS und gegen TNF α die Überlebensrate erhöht werden kann (WO 91-01755).

Antikörper-Therapieverfahren weisen jedoch schwerwiegende Mängel und Nachteile auf. Die Kosten für die technisch aufwendige Gewinnung, Reinigung und Charakterisierung der entsprechenden Antikörper sind sehr hoch und es besteht die Gefahr der allergischen Gegenreaktion (neutralisierende Immunantwort) des Körpers auf die Antikörper. Bezüglich der LPS-Antikörper können die hohen Raten von Therapieversagen unter anderem auf die zu geringe Spezifität bzw. Affinität zwischen den sehr heterogenen LPS-Molekülen und den verwendeten mono- bzw. polyklonalen Antikörpern zurückgeführt werden. In diesem Zusammenhang mußten klinische Multi-Zentren-Studien vorzeitig abgebrochen werden (Luce, J.M., Crit. Care Med. 21, 1993, 1233 ff).

Eine andere Vorgehensweise zur.Neutralisation bzw. Elimination pathogener Blutbestandteile besteht in der Behandlung von Vollblut oder Plasma in einem extrakorporalen Perfusionssystem unter Verwendung entsprechend geeigneter Adsorbermaterialien

Folgende Adsorbermaterialien wurden für eine extrakorporale Elimination von Lipopolysacchariden (LPS, Endotoxine) aus Vollblut und/oder Plasma als potentiell geeignet offenbart: Poröse Trägermaterialien mit immobilisiertem Polymyxin B (US 4, 576, 928; DE 3932 971). Die klinische Anwendung dieser Affinitätsträger ist jedoch sehr problematisch, da der Ligand Polymyxin B schwere nephro- und neurotoxische Schäden bei seiner Freisetzung in die Blutbahn hervorruft.

Die in DE 41 13 602 A1 offenbarten polyethylenimin-modifizierten Perlcellulosen besitzen eine geringe Bindungskapazität für LPS. Bei ihrer Verwendung in einem extrakorporalen Perfusionssystem würde daher das medizinisch-tolerierbare extrakorporale Totvolumen überschritten.

Für die extrakorporale Adsorptionsapherese von Tumor-Nekrose-Faktor α und/oder LPS aus Vollblut und/oder Plasma werden in DE 43 313 58 A1 Polyanionen-modifizierte Trägermaterialien offenbart. Ein Nachteil dieser Kationenaustauscher-Materialien liegt darin, daß sie bezüglich LPS eine zu geringe Selektivität und Effektivität aufweisen und nur ca. 30 % der im perfundierten Plasma vorhandenen Lipopolysaccharide adsorbieren bzw. eliminieren. Dies ist verständlich, da bakterielle Lipopolysaccharide bei physiologischem pH-Wert als negativ geladene Moleküle vorliegen und damit eine geringe Bindungsaffinität bezüglich Kationenaustauscher-Materialien aufweisen. Um den klinischen Verlauf des septischen Krankheitsbildes günstig zu beeinflussen, ist es jedoch - wie bereits ausgeführt - aus pathophysiologischer und therapeutischer Sicht wünschenswert, beide pathogenen Blutbestandteile (LPS und TNF α) nicht nur gleichzeitig, sondern auch mit hoher Effektivität aus dem Kreislauf des Patienten zu entfernen. Durch diese Maßnahme wird die biologische Mediatorkaskade initial unterbrachen und die fatalen synergistischen Effekte der beiden Pathogene TNF α und LPS wirkungsvoll ausgeschaltet.

Die Aufgabe der vorliegenden Erfindung war es daher, ein Verfahren bereitzustellen, um bakterielle Lipopolysaccharide (LPS) und Tumor-Nekrose-Faktor α (TNF α) gleichzeitig und mit hoher Effektivität aus Vollblut oder/und Blutplasma in einem extrakorporalen Perfusionssystem zu entfernen.

Um ein derartiges Eliminationsverfahren (Adsorptionsapherese) nutzen zu können, müssen u.a. folgende Voraussetzungen erfüllt sein:
1) Die Elimination der Pathogene sollte möglichst selektiv und effizient erfolgen.
2) Die Bindungskapazität der verwendeten Adsorbentien sollte optimalen praktischen Anforderungen genügen.
3) Die Adsorbentien müssen ohne Verlust oder Veränderung ihrer Eigenschaften mit Hitze oder gamma-Strahlen sterilisierbar sein.
4) Die Adsorbentien sollten eine ausreichend hohe Durchflußgeschwindigkeit im Bereich bis zu 200 ml/min erlauben.
5) Das Eliminationsverfahren muß die medizinisch-erforderliche Bio- bzw. Hämokompabilität aufweisen und darf keine physiologischen Regelkreise und Schutzmechanismen, wie beispielsweise das Immun-, Komplement- oder Gerinnungssystem, beeinträchtigen.

Gelöst wird diese Aufgabe erfindungsgemäß durch ein Verfahren zur extrakorporalen Entfernung von Tumor-Nekrose-Faktor α (TNF α) oder/und bakteriellen Lipopolysacchariden (LPS, Endotoxin) aus Vollblut oder/und Blutplasma in einem extrakorporalen Perfusionssystem, bei dem man das Blut oder Plasma über ein Kationenaustauscher- und ein Anionenaustauschermaterial leitet.

Im Rahmen der Erfindung ist es hierbei bevorzugt, Kationenaustauscher- und Anionenaustauschermaterialien gemischt, sozusagen als Mischbett zu verwenden. Weiterhin ist es bevorzugt, bifunktionale Ionenaustauschermaterialien zu verwenden; d.h.

Materialien, die aufgrund der enthaltenen Gruppierungen fähig sind, sowohl Anionen als auch Kationen zu binden. Derartige Materialien und ihre Herstellung sind dem Fachmann geläufig.

Im Rahmen der vorliegenden Erfindung verwendet man vorteilhaft Ionenaustauschermaterialien mit Trägermaterialien aus porösem Glas oder/und mit organischen Polymerisaten oder Copolymerisaten beschichtetem Kieselgel, quervernetzten Kohlehydraten oder/und organischen Polymerisaten oder Copolymerisaten in Form von porösen Partikeln oder mikroporösen Membran- oder/und Hohlfaserstrukturen.

Ein erfindungsgemäß besonders geeignetes Kationenaustauschermaterial besteht aus einem Trägermaterial mit daran kovalent gebundenen funktionellen Gruppen aus synthetischen oder/und halbsynthetischen oder/und natürlichen Polyanionenketten, und zwar in linearer oder verzweigter Form. Werden poröse Trägermaterialien verwendet, so sind diese vorzugsweise so beschaffen, daß sie einen mittleren Porendurchmesser von < 30 nm oder/und eine molekulare Ausschlußgröße für globuläre Proteine von < 10⁶ und insbesondere < 2 x 10⁴ Dalton aufweisen. Die Polyanionenketten haben dabei wiederum besonders bevorzugt ein mittleres Molekulargewicht von 600 bis 10⁶ Dalton, insbesondere 5 x 10³ bis 5 x 10⁵ Dalton. Natürliche Polyanionenketten bestehen im erfindungsgemäßen Verfahren vorzugsweise aus biologischen Polycarbon- oder/und Polysulfonsäuren und besonders geeignet sind sulfatierte Polysaccharide.

Bevorzugte synthetische bzw. halbsynthetische Polyanionenketten sind Polymerisate oder Copolymerisate der Monomere Acrylsäure, Methacrylsäure, Vinylsulfonsäure, Maleinsäure; Acryl- oder/und Methacrylsäurederivate der Formel H₂C=CR₁-CO-R₂, wobei der Substituent R₁ Wasserstoff oder eine Methylgruppe und R₂ eine amid- oder esterartig gebundene lineare oder/und verzweigtkettige aliphatische Sulfonsäure-, Carbonsäure- oder/und Phosphorsäuregruppe ist; Styrolsulfonsäure, Anetolsulfonsäure, Styrolphosphorsäure; Glutaminsäure, Asparaginsäure; Adenosin-3',5'diphosphat, Guanosin-3',5'-diphosphat. Ganz besonders bevorzugt wird als Kationenaustauschermaterial im Rahmen der vorliegenden Erfindung Dextransulfatcellulose verwendet.

Als Anionenaustauscher verwendet man im Rahmen des erfindungsgemäßen Verfahrens vorzugsweise Materialien, welche als funktionelle Gruppen Kationen oder natürliche, synthetische oder halbsynthetische Polykationenketten an Trägermaterialien fixiert enthalten, wobei Polykationenketten in linearer oder verzweigter Form vorliegen können. Besonders bevorzugt verwendet man als Kationen- bzw. Polykationenketten tertiäre oder/und quartäre Amine.

Bevorzugte Anionenaustauschermaterialien schließen dabei quervernetzte oder/und mikrogranuläre oder/und mikroporöse Dialkylaminoalkyl-, Dialkylaminoaryl-, Trialkylammoniumalkyloder Trialkylammoniumaryl-Cellulosen oder/und Dialkylaminoalkyl-, Dialkylaminoaryl-, Trialkylammoniumalkyl- oder Trialkylammoniumaryl-modifizierte organische Polymere oder Copolymere ein.

Überraschenderweise wurde im Rahmen der vorliegenden Erfindung festgestellt, daß erfindungsgemäße Anionenaustauscher, wofür der Heparinadsorber 500 (B. Braun Melsungen AG, Melsungen) ein besonders bevorzugtes Beispiel ist, bakterielle Lipopolysaccharide bei physiologischem pH-Wert (pH 7.4) mit hoher Selektivität und Kapazität (> 3 mg LPS/g Trockengewicht) adsorptiv aus Vollblut oder/und Blutplasma binden bzw. eliminieren (Beispiel 1).

Das erfindungsgemäße Verfahren wird vorzugsweise bei physiologischem pH-Wert durchgeführt.

Weiterhin wurde überraschenderweise festgestellt, daß derartige Anionenaustauscher auch bei physiologischem pH-Wert nur eine geringe und von der Zusammensetzung unschädliche Menge an Blut- bzw. Plasmaproteinen adsorbieren (Beispiel 3).

Das erfindungsgemäße Verfahren kann durch geführt werden in einer Vorrichtung zur extrakorporalen Behandlung von Patientenblut oder Plasma, welche ein Kationenaustauscher- und ein Anionenaustauschermaterial enthält, wobei diese Materialien in mindestens einer Abteilung eines extrakorporalen Perfusionssystems enthalten sind. Es werden dabei zwei getrennte Abteilungen oder Kartuschen in der Vorrichtung vorliegen, die zum einen mit dem Anionenaustauschermaterial zum Entfernen von LPS und zum anderen mit dem Kationenaustauschermaterial zur Entfernung von TNF α gefüllt sind. Diese beiden Abteilungen sind dann über entsprechende Aus- bzw. Einlaßöffnungen so miteinander verbunden, daß das Patientenblut oder -plasma gleichmäßig durch beide Abteilungen geleitet wird. Dies geschieht zweckmäßigerweise mit Hilfe direkter Schlauchverbindungen. Andere Verbindungsarten sind jedoch ebenfalls möglich. In einer anderen möglichen Form liegen das Anionen- und Kationenaustauschermaterial in einer einzigen Abteilung in Form eines Mischbetts, wobei die Materialien in einem den praktischen Anforderungen entsprechenden Verhältnis miteinander vermischt und abgefüllt sind ohne Beeinträchtigung ihrer vorteilhaften Eigenschaften, oder in Form bivalenter Ionenaustauschermaterialien vor. Diese Ausgestaltung des erfindungsgemäßen Verfahrens ist besonders kostengünstig, einfach und sicher zu handhaben, da insbesondere auch extrakorporales Perfusionsvolumen eingespart wird und die zu sterilisierenden und steril zu haltenden Verbindungen und Teilstücke der Vorrichtung in geringer Anzahl gehalten werden können.

Vollblut wird mit Hilfe einer Schlauchpumpe entweder direkt über die kombinierten bzw. gemischten Adsorbermaterialien geleitet, oder es wird zunächst über einen entsprechenden Separator (Membranfilter, Hohlfasermembran, Durchflußzentrifuge) von den zellulären Bestandteilen getrennt. Das so erhaltene Plasma wird über die erfindungsgemäß kombinierten bzw. gemischten Adsorbermaterialien geleitet, von den Pathogenen befreit, anschließend mit den zellulären Blutkomponenten vereinigt und dem Patienten zurückgegeben.

Erfindungsgemäß ist es daher bevorzugt, daß der oder den Abteilungen mit den Ionenaustauschermaterialien der Vorrichtung eine Plasmaseparationseinheit vorgeschaltet ist. Diese Plasmaseparationseinheit besteht vorzugsweise aus einem Plasmafraktionierungsfilter, der für Fibrinogen- und/oder Low Density-Lipoproteine (LDL) impermeabel ist.

Die Abteilungen der Vorrichtung, in denen die Ionenaustauschermaterialien enthalten sind, werden vorzugsweise jeweils von zylindrischen Gehäusen gebildet, die an den stirnseitigen Enden mit Deckeln versehen sind, die jeweils einen zentralen Zu- und Ablaufstutzen aufweisen. Die Gehäuse haben dabei vorzugsweise einen Durchmesser von 3 bis 20 cm, insbesondere 5 bis 10 cm und eine Länge von 1 bis 40 cm, insbesondere 5 bis 20 cm. Die Gehäuse sind desweiteren vorzugsweise aus Glas oder Kunststoff, wobei in den Deckeln der Gehäuse vorzugsweise Siebe mit 10 bis 200 µm, insbesondere 20 bis 100 µm Porenweite zur Eliminierung von Partikeln integriert sind. Für die Vorrichtung ist es desweiteren bevorzugt, daß die Gehäuse in einem geschlossenen Kreislauf integriert sind, in dem das Vollblut bzw. Blutplasma mittels einer Pumpe zirkuliert wird.

Die Trägermaterialien der in der Vorrichtung enthaltenen Kationen- und Anionenaustauschermaterialien bestehen vorzugsweise aus porösem Glas oder/und mit organischen Polymerisaten oder Copolymerisaten beschichtetem Kieselgel, quervernetzten Kohlehydraten oder/und organischen Polymerisaten oder Copolymerisaten in Form von porösen Strukturen, wie z.B. Partikeln, oder mikroporösen Membran- oder/und Hohlfaserstrukturen.

Das in der Vorrichtung enthaltene Kationenaustauschermaterial besteht vorzugsweise aus einem Trägermaterial mit daran kovalent gebundenen funktionellen Gruppen aus synthetischen oder/und halbsynthetischen oder/und natürlichen Polyanionenketten, und zwar in linearer oder verzweigter Form.

Die Polyanionenketten des Kationenaustauschers weisen vorzugsweise ein mittleres Molekulargewicht von 600 bis 10⁶ Dalton, insbesondere 5 x10³ bis 5 x 10⁵ Dalton auf. Wird ein poröses Trägermaterial verwendet, so ist der mittlere Porendurchmesser des Kationenaustauschermaterials erfindungsgemäß bevorzugt < 30 nm oder/und die molekulare Ausschlußgrenze für globuläre Proteine < 10⁶, insbesondere < 2 x 10⁴ Dalton. Es ist hierbei bevorzugt, daß natürliche Polyanionenketten des Kationenaustauschers aus biologischen Polycarbon- oder/und Polysulfonsäuren, insbesondere aus sulfatierten Polysacchariden bestehen, und daß synthetische bzw. halbsynthetische Polyanionenketten Polymerisate oder Copolymerisate der Monomere Acrylsäure, Methacrylsäure, Vinylsulfonsäure, Maleinsäure; Acryl- oder/und Methacrylsäurederivate der Formel H₂C=CR₁-CO-R₂, wobei der Substituent R₁ Wasserstoff oder eine Methylgruppe und R₂ eine amidoder esterartig gebundene lineare oder/und verzweigtkettige aliphatische Sulfonsäure-, Carbonsäure- oder/und Phosphorsäuregruppe ist; Styrolsulfonsäure, Anetolsulfonsäure, Styrolphosphorsäure; Glutaminsäure, Asparaginsäure; Adenosin-3',5'diphosphat, Guanosin-3',5'-diphosphat sind.

Die in der Vorrichtung enthaltenen Anionenaustauscher sind vorzugsweise Materialien, welche als funktionelle Gruppen Kationen oder natürliche, synthetische oder halbsynthetische Polykationenketten an Trägermaterialien fixiert enthalten, wobei Polykationenketten in linearer oder verzweigter Form vorliegen können. Bevorzugt sind als Kationen- bzw. Polykationen tertiäre oder/und quartäre Amine.

Ein besonders bevorzugtes Kationenaustauschermaterial ist Dextransulfatcellulose und das Anionenaustauschermaterial umfaßt bevorzugt quervernetzte oder/und mikrogranuläre oder/und mikroporöse Dialkylaminoalkyl-, Dialkylaminoaryl-, Triälkylammoniumalkyl- oder Trialkylammoniumaryl-Cellulosen oder/und Dialkylaminoalkyl-, Dialkylaminoaryl-, Trialkylammoniumalkyloder Trialkylammoniumaryl-modifizierte organische Polymere oder Copolymere.

Das erfindungsgemäße extrakorporale Adsorptionsapherese-Verfahren zur simultanen Elimination von LPS und TNFα erweist sich dahingehend als vorteilhaft, daß
1) das Verfahren einfach zu überwachen und sicher zu handhaben ist,
2) das extrakorporale Totvolumen gering ist,
3) das Blut- bzw. Blutplasma unter physiologischen pH-Bedingungen behandelt werden kann,
4) bei Verwendung des Mischbett-Adsorbers das behandelte Blut- bzw. Blutplasma einer geringeren Fremdoberfläche ausgesetzt ist,
5) bei Verwendung des Mischbett-Adsorbers der Aufwand an Apparaturen und Schlauchleitungen (Einmalartikel) vereinfacht und somit wirtschaftlicher ist.

Zusammenfassend ermöglicht das erfindungsgemäße Verfahren erstmals, auf einfache, selektive und effektive Weise die beiden Hauptmediatoren (bakterielle Lipopolysaccharide, Tumor-Nekrose-Faktor α) der septischen Krankheitszustände aus Patientenblut unter physiologischen pH-Bedingungen in einem apparativ einfach ausgestatteten extrakorporalen Perfusionssystem zu entfernen. Die folgenden Beispiele erläutern die Erfindung weiter.

### Beispiel 1

### Vorrichtung und Verfahren zur simultanen Elimination von Lipopolysaccharid (LPS) und Tumor-Nekrose-Faktor α (TNF α) aus Humanplasma

### Versuchsaufbau

Eine mit dem erfindungsgemößen Kationenaustauscher-Material gefüllte Kartusche (Bettvolumen: 160 ml; Liposorber™ LA-15, Kanegafuchi Chemical Industry, Osaka, Japan) wurde über die Auslaßöffnung mit Hilfe einer direkten Schlauchverbindung zur Einlaßöffnung einer mit dem erfindungsgemäßen Anionenaustauscher-Material gefüllten Kartusche (Bettvolumen: 500 ml; Heparinadsorber 500, B. Braun Melsungen AG, Melsungen) gekoppelt.

### Versuchsdurchführung

Die beiden gekoppelten Adsorber wurden zunächst mit 6 1 einer pyrogenfreien Lösung (Ringerlösung bestehend aus 140 mmol/l NaCl, 2 mmol/l CaCl₂ und 4 mmol/l KCl) konditioniert (Flußrate: 100/min). Unter sterilen Bedingungen wurden 1200 ml frisch gewonnenes Humanplasma mit 205 EU/ml (14.7 ng/ml) an bakteriellem Lipopolysaccharid (E. coli 055: B5 Endotoxin; Fa. BioWhittaker, Walkersville, USA) und mit 450 ng/ml an Tumor-Nekrose-Faktor α (TNF α, Fa. Serva, Heidelberg) versetzt.

Anschließend wurde das Humanplasma mit einer Schlauchpumpe (Flußrate: 30 ml/min) über die beiden in Serie geschalteten Adsorber gepumpt.

Die quantitative Bestimmung des Lipopolysaccharids erfolgte mit Hilfe des chromogenen, kinetischen Limulus-Amoebocyten-Lysat (LAL) Tests (Fa. Chromogenix AB, Mölndal, Schweden). Der Tumor-Nekrose-Faktor α wurde mit Hilfe eines EAISA (Enzyme Amplified Sensitivity Immunoassay; Fa. Medgenix Diagnostics SA, Fleurus, Belgien) quantifiziert.

### Versuchsergebnisse:

Die quantitative Bestimmung von LPS und TNF α in dem erfindungsgemäß perfundierten Humanplasma ergab, daß 70 % des zugesetzten TNF α und 98 % des zugesetzten LPS durch Adsorption bei physiologischem pH-Wert eliminiert wurden.

### Beispiel 2

### Adsorption und Elimination von Lipopoysaccharid (LPS, Endotoxin) aus Humanplasma unter physiologischen pH-Bedingungen

1200 ml frisch gewonnenes Humanplasma wurden mit 205 EU/ml (14,7 ng/ml) an bakteriellem Lipopolysaccharid (E. coli 055:B5 Endotoxin, Fa. BioWhittaker, Walkersville, USA) versetzt und mit einer Flußrate von 30 ml/min über einen mit 6000 ml einer pyrogenfreien physiologischen Kochsalzlösung konditionierten Heparin-Adsorber 500 (Fa. B. Braun, Melsungen) gepumpt. Die quantitative Bestimmung des Lipopolysaccharids (chromogener, kinetischer Limulus-Amoebocyten-Lysat Test, Fa. Chromogenix AB, Mölndal, Schweden) im perfundierten Eluat ergab, daß 96 % des zugesetzten Lipopolysaccharids (Endotoxin) durch Bindung an den Adsorber eliminiert wurden.

### Beispiel 3

### Adsorption von funktionellen und katalytischen Plasmaproteinen bei Perfusion von Humanplasma unter physiologischen pH-Bedingungen über den Heparin-Adsorber 500

Nach der Perfusion (Flußrate: 30 ml/min) von 1000 ml frisch gewonnenem Humanplasma über einen mit 6000 ml physiologischer Kochsalzlösung konditionierten Heparin-Adsorber 500 (Fa. B. Braun, Melsungen) wird die Adsorber-Kartusche mit 2000 ml einer physiologischen Kochsalzlösung gespült (Flußrate: 30 ml/min). Anschließend wird die Flußrichtung umgekehrt und die Kartusche für 30 min bei einer Flußrate von 100 ml/min rezirkulierend mit 300 ml einer 2 M Natriumchlorid-Lösung gespült, um die durch Adsorption gebundenen Plasmaproteine zu eluieren.

Die Tabelle 1 zeigt, daß bei der - erfindungsgemäß - unter physiologischen pH-Bedingungen erfolgten Perfusion von 1000 ml Humanplasma über den Heparin-Adsorber 500 nur 3.1 % des Gesamtprotein-Gehalts des unbehandelten Plasmas adsorptiv gebunden wurden.

Die quantitative Bestimmung der verschiedenen - in Tabelle 1 aufgeführten - Plasmaproteine ergab, daß nur vier Proteine in einem signifikanten Umfang eliminiert wurden. Bezüglich dieser Proteine (Retinol bindendes Protein, Coeruloplasmin, Präalbumin, IgM) ist jedoch bekannt, daß eine körpereigene Substitution sehr rasch erfolgt und eine zeitlich begrenzte Reduktion keine unerwünschten physiologischen Reaktionen hervorruft.

Die quantitative Bestimmung der Plasmaenzyme GPT, GOT, AP, α-Amylase, GT, GLDH, CK, LDH, CHE und Lipase ergab, daß keines der untersuchten Enzyme adsorptiv gebunden und dadurch eliminiert wurde.

**Tabelle 1**

| Adsorption von funktionellen Plasmaproteinen an den Heparin-Adsorber 500 (Fa. B. Braun, Melsungen) bei physiologischem pH-Wert | | |
|---|---|---|
| | [mg]^{a} | [%]^{b} |
| Gesamtprotein | 2250 | 3.9 |
| Albumin | 750 | 2.1 |
| Präalbumin | 195 | 80.3 |
| IgA | 83 | 4.2 |
| IgG | 182 | 1.8 |
| IgM | 184 | 32.3 |
| Fibrinogen | 147 | 7.1 |
| β₂-Mikroglobulin | 0.015 | 1.6 |
| α₂-Makroglobulin | 57 | 3.2 |
| Coeruloplasmin | 110 | 60.0 |
| Haptoglobin | 102 | 9.0 |
| Haemopexin | 50 | 7.4 |
| Retinol bind. Protein | 24 | 64.9 |
| Ferritin | 0.002 | 6.9 |
| Transferrin | 94 | 5.2 |
| α_{*1*}-Antitrypsin | 58 | 3.9 |
| α_{*1*}-Glycoprotein | 50 | 9.1 |

| | | |
|---|---|---|
| ^{a} bezogen auf 1000 ml perfundiertes Humanplasma | | |
| ^{b} prozentualer Anteil gegenüber dem unbehandelten Humanplasma | | |

## Patentansprüche

1. Verfahren zur extrakorporalen Entfernung von Tumor-Nekrose-Faktor α (TNF α) oder/und bakteriellen Lipopolysacchariden (LPS, Endotoxin) aus Vollblut oder/und Blutplasma in einem extrakorporalen Perfusionssystem, bei dem man das Blut oder Plasma über ein Kationenaustauscher- und ein Anionenaustauschermaterial leitet.

2. Verfahren nach Anspruch 1,
**dadurch gekennzeichnet,**
**daß** das Kationenaustauscher- und das Anionenaustauschermaterial als Mischbett oder als bifunktionaler lonenaustauscher vorliegen.

3. Verfahren nach Anspruch 1 oder 2,
**dadurch gekennzeichnet,**
**daß** man Kationen- oder/und Anionenaustauscher mit Trägermaterialien aus porösem Glas oder/und mit organischen Polymerisaten oder Copolymerisaten beschichtetem Kieselgel, quervernetzten Kohlehydraten oder/und organischen Polymerisaten oder Copolymerisaten in Form von porösen Partikeln oder mikroporösen Membran- oder/und Hohlfaserstrukturen verwendet.

4. Verfahren nach Anspruch 1, 2 oder 3,
**dadurch gekennzeichnet,**
**daß** das Kationenaustauschermaterial aus einem Trägermaterial mit daran kovalent gebundenen funktionellen Gruppen aus synthetischen oder/und halbsynthetischen oder/und natürlichen Polyanionenketten, und zwar in linearer oder verzweigter Form, besteht.

5. Verfahren nach Anspruch 4,
**dadurch gekennzeichnet,**
**daß** man poröse Trägermaterialien mit einem mittleren Porendurchmesser von < 30 nm oder/und einer molekularen Ausschlußgrenze für globuläre Proteine von < 10⁶, insbesondere < 2 x 10⁴ Dalton verwendet.

6. Verfahren nach Anspruch 4 oder 5,
**dadurch gekennzeichnet,**
**daß** man Polyanionenketten mit einem mittleren Molekulargewicht von 600 bis 10⁶ Dalton, insbesondere 5 x 10³ bis 5 x 10⁵ Dalton verwendet.

7. Verfahren nach einem der Ansprüche 4 bis 6,
**dadurch gekennzeichnet,**
**daß** man natürliche Polyanionenketten aus biologischen Polycarbonoder/und Polysulfonsäuren und insbesondere aus sulfatierten Polysacchariden verwendet.

8. Verfahren nach einem der Ansprüche 4 bis 7,
**dadurch gekennzeichnet,**
**daß** man als synthetische bzw. halbsynthetische Polyanionenketten Polymerisate oder Copolymerisate der Monomere Acrylsäure, Methacrylsäure, Vinylsulfonsäure, Maleinsäure; Acryl- oder/und Methacrylsäurederivate der Formel H₂C=CR₁-CO-R₂, wobei der Substituent R₁ Wasserstoff oder eine Methylgruppe und R₂ eine amidoder esterartig gebundene lineare oder/und verzweigtkettige aliphatische Sulfonsäure-, Carbonsäure-oder/und Phosphorsäuregruppe ist; Styrolsulfonsäure, Anetolsulfonsäure, Styrolphosphorsäure; Glutaminsäure, Asparaginsäure; Adenosin-3',5'-diphosphat, Guanosin-3',5'-diphosphat verwendet.

9. Verfahren nach einem der Ansprüche 4 bis 8,
**dadurch gekennzeichnet,**
**daß** man als Kationenaustauschermaterial Dextransulfat-Cellulose verwendet.

10. Verfahren nach einem der vorhergehenden Ansprüche,
**dadurch gekennzeichnet,**
**daß** man als Anionenaustauscher Materialien verwendet, welche als funktionelle Gruppen Kationen oder natürliche, synthetische oder halbsynthetische Polykationenketten an Trägermaterialien fixiert enthalten, wobei Polykationenketten in linearer oder verzweigter Form vorliegen können.

11. Verfahren nach Anspruch 10,
**dadurch gekennzeichnet,**
**daß** man als Kationen bzw, Polykationen tertiäre oder/und quartäre Amine verwendet.

12. Verfahren nach einem der vorhergehenden Ansprüche,
**dadurch gekennzeichnet**
**daß** man als Anionenaustauschermaterial quervernetzte oder/und mikrogranuläre oder/und mikroporöse Dialkylaminoalkyl-, Dialkylaminoaryl-, Trialkylammoniumalkyl- oder Trialkylammoniumaryl-Cellulosen oder/und Dialkylaminoalkyl-, Dialkylaminoaryl-, Trialkylammoniumalkyl- oder Trialkylammonlumaryl-modifizierte organische Polymere oder Copolymere verwendet.

13. Verfahren nach einem der vorhergehenden Ansprüche,
**dadurch gekennzeichnet,**
**daß** es bei physiologischem pH-Wert durchgeführt wird.

## Claims

1. Process for the extracorporeal removal of tumour necrosis factor α (TNF α) or/and bacterial lipopolysaccharides (LPS, endotoxin) from whole blood or/and blood plasma in an extracorporeal perfusion system, in which the blood or plasma is passed over a cation exchanger material and an anion exchanger material.

2. Process as claimed in claim 1,
**wherein**
the cation exchanger material and the anion exchanger material are present as a mixed bed or as a bifunctional ion exchanger.

3. Process as claimed in claim 1 or 2,
wherein
cation or/and anion exchangers with support materials composed of porous glass or/and silica gel coated with organic polymers or copolymers, cross-linked carbohydrates or/and organic polymers or copolymers in the form of porous particles or microporous membrane or/and hollow fibre structures are used.

4. Process as claimed in claim 1, 2 or 3,
**wherein**
the cation exchanger material is composed of a support material to which functional groups made of synthetic or/and semisynthetic or/and natural polyanion chains are bound covalently and namely in a linear or branched form.

5. Process as claimed in claim 4,
wherein
porous support materials with an average pore diameter of < 30 nm or/and a molecular exclusion limit for globular proteins of < 10⁶ and in particular < 2 x 10⁴ daltons are used.

6. Process as claimed in claim 4 or 5,
**wherein**
polyanion chains with an average molecular weight of 600 to 10⁶ daltons and in particular 5 x 10³ to 5 x 10⁵ daltons are used.

7. Process as claimed in one of the claims 4 to 6,
wherein
natural polyanion chains made of biological polycarboxylic or/and polysulfonic acids and in particular of sulfated polysaccharides are used.

8. Process as claimed in one of claims 4 to 7,
wherein
polymers or copolymers of the monomers acrylic acid, methacrylic acid, vinylsulfonic acid, maleic acid; acrylic acid derivatives or/and methacrylic acid derivatives of the formula H₂C=CR₁-CO-R₂ in which the substituent R₁ is hydrogen or a methyl group and R₂ is a linear or/and branched-chain aliphatic sulfonic acid, carboxylic acid or/and phosphoric acid group bound in an amide-like or ester-like manner; styrenesulfonic acid, anetholesulfonic acid, styrenephosphoric acid; glutamic acid, aspartic acid; adenosine-3',5'-diphosphate, guanosine-3',5'-diphosphate are used as the synthetic or semisynthetic polyanion chains.

9. Process as claimed in one of the claims 4 to 8,
wherein
dextran sulfate cellulose is used as the cation exchanger material.

10. Process as claimed in one of the previous claims,
wherein
materials are used as the anion exchanger which contain cations or natural, synthetic or semisynthetic polycation chains as functional groups attached to support materials, in which the polycation chains can be present in a linear or branched form.

11. Process as claimed in claim 10,
wherein
tertiary or/and quaternary amines are used as the cations or polycations.

12. Process as claimed in one of the previous claims,
wherein
cross-linked or/and microgranular or/and microporous dialkylaminoalkyl, dialkylaminoaryl, trialkylammoniumalkyl or trialkylammoniumaryl celluloses or/and dialkylaminoalkyl, dialkylaminoaryl, trialkylammoniumalkyl or trialkylammonlumaryl-modified organic polymers or copolymers are used as the anion exchanger material.

13. Process as claimed in one of the previous claims,
wherein
it is carried out at a physiological pH value.

## Revendications

1. Procédé pour l'élimination extracorporelle du facteur de nécrose tumorale alpha (TNF alpha) ou/et de lipopolysaccharides (LPS, endotoxine) à partir du sang complet ou/et du plasma sanguin dans un système de perfusion extracorporel, dans lequel on conduit le sang ou le plasma par un matériau échangeur de cations et échangeur d'anions.

2. Procédé selon la revendication 1,
**caractérisé en ce que**,
le matériau échangeur de cations et échangeur d'anions se présente en tant que lit mélangé ou en tant qu'échangeur d'ions bifonctionnel.

3. Procédé selon la revendication 1 ou 2,
**caractérisé en ce que**,
l'on utilise un échangeur de cations ou/et d'anions avec des supports en verre poreux ou/et avec du gel de silice revêtu de polymérisats ou copolymérisats organiques, des hydrates de carbone réticulés ou/et des polymérisats ou des copolymérisats organiques sous la forme de particules poreuses ou de structures membranaires microporeuses ou/et de fibres creuses.

4. Procédé selon la revendication 1,2 ou 3,
**caractérisé en ce que**,
le matériau échangeur de cations consiste en un support avec des groupes fonctionnels liés par covalence à celui-ci en chaînes de polyanions synthétiques ou/et semi-synthétiques ou/et naturels et ce, sous la forme linéaire ou ramifiée.

5. Procédé selon la revendication 4,
**caractérisé en ce que**,
l'on utilise des supports poreux ayant un diamètre de pore moyen < 30 nm ou/et avec une limite d'exclusion moléculaire pour les protéines globulaires < 10⁶, en particulier < 2 x 10⁴ daltons.

6. Procédé selon la revendication 4 ou 5,
**caractérisé en ce que**,
l'on utilise des chaînes de polyanions ayant un poids moléculaire moyen de 600 à 10⁶ daltons, en particulier 5 x 10³ jusqu'à 5 x 10⁵ daltons.

7. Procédé selon l'une des revendications 4 à 6,
**caractérisé en ce que**,
l'on utilise des chaînes de polyanions naturelles à partir d'acides biologiques polycarboxyliques ou/et polysulfoniques et en particulier, à partir de polysaccharides sulfatés.

8. Procédé selon l'une des revendications 4 à 7,
**caractérisé en ce que**,
l'on utilise en tant que chaînes de polyanions synthétiques ou semi-synthétiques des polymérisats ou copolymérisats des monomères acide acrylique, acide méthacrylique, acide vinylsulfonique, acide maléinique; dérivés de l'acide acrylique ou/et méthacrylique de la formule H₂C=CR₁-CO-R₂, tandis que le substituant R₁ est l'hydrogène ou un groupe méthyle et R₂ est un groupe de l'acide phosphorique ou/et de l'acide carboxylique, de l'acide sulfonique aliphatique à chaîne ramifiée ou linéaire lié à la manière d'un ester ou d'un amide; l'acide styrolsulfonique, l'acide anétolsulfonique, l'acide styrolphosphorique, l'acide glutaminique, l'acide asparaginique, l'adénosin-3',5'-diphosphate, le guanosin-3',5'-diphosphate.

9. Procédé selon l'une des revendications 4 à 8,
**caractérisé en ce que**,
l'on utilise la dextransulfate-cellulose en tant que matériau échangeur de cations.

10. Procédé selon l'une des revendications précédentes,
**caractérisé en ce que**,
en tant qu'échangeur d'anions on utilise des matériaux qui contiennent comme groupes fonctionnels des cations ou des chaînes de polycations naturels, synthétiques ou semi-synthétiques fixées sur les supports où les chaînes de polycations peuvent se présenter sous la forme linéaire ou ramifiée.

11. Procédé selon la revendication 10,
**caractérisé en ce que**,
en tant que cations ou polycations, on utilise des amines tertiaires ou/et quaternaires.

12. Procédé selon l'une des revendications précédentes,
**caractérisé en ce que**,
en tant que matériau échangeur d'anions, on utilise des celluloses de dialkylaminoalkyle, de dialkylaminoaryle, de trialkylammoniumalkyle ou de trialkylammoniumaryle réticulées ou/et microgranulaires ou/et microporeuses ou des polymères ou copolymères organiques dialkylaminoalkyle, dialkylaminoaryle, triakylammoniumalkyle ou trialkylammoniumaryle modifiés.

13. Procédé selon l'une des revendications précédentes,
**caractérisé en ce qu'**il est ajusté à une valeur de pH physiologique.
